# EUROPEAN PATENT SPECIFICATION

(11) **EP 4 114 508 B1**
(45) Date of publication and mention of the grant of the patent: **17.09.2025**
(21) Application number: 21710079.1
(22) Date of filing: 23.02.2021
(51) Int. Cl.: A61N 1/30, A61N 1/32, A61K 9/00, A61M 5/00, G01N 33/483

(54) **A SYSTEM AND METHOD FOR RELEASING A SPECIES**
SYSTEM UND VERFAHREN ZUR FREISETZUNG EINER SPEZIES
SYSTÈME ET PROCÉDÉ POUR LIBÉRER UNE ESPÈCE

(30) Priority: 24.02.2020 SE 2050204
(43) Date of publication of application: 11.01.2023
(73) Proprietor: OBOE IPR AB, 581 18 Linköping (SE)
(72) Inventor: BINTINGER, Johannes, 602 40 Norrköping (SE); MIKULA, Hannes, 3441 Pixendorf (AT); SIMON, Daniel, 582 25 Linköping (SE); BERGGREN, Magnus, 602 18 Norrköping (SE)
(74) Representative: Zacco Sweden AB
(86) International application number: PCT/SE2021/050150
(87) International publication number: WO 2021/173062

(56) References cited:
- WO-A1-2017/044983
- WO-A1-2018/187740
- US-A1- 2019 111 251
- US-B2- 9 694 177
- DANIEL T SIMON ET AL: "Precise Neurotransmitter-Mediated Communication with Neurons In Vitro and In Vivo Using Organic Electronics", JOURNAL OF BIOMECHANICAL SCIENCE AND ENGINEERING, 1 January 2010 (2010-01-01), pages 208 - 217, XP055373148, Retrieved from the Internet <URL:https://www.jstage.jst.go.jp/article/jbse/5/3/5_3_208/_pdf> DOI: 10.1299/jbse.5.208

## Description

### TECHNICAL FIELD

The present disclosure relates to a system and method for transporting a first species from a source solution through an ion conductive member to a target solution, wherein the first species is arranged to interact with the second species in the target solution, such that at least one part of the second species is released.

### BACKGROUND

Presently, transport of charged species from, to or between solutions, such as from a stock solution to a cell culture medium, is performed by manual or automated use of e.g. pipettes, pumps or membranes. Such techniques result in unspecific delivery of charged species to a cell culture medium as such only, whereas further diffusion to cells cultured in the medium is uncontrollable and unpredictable.

Controlling the amount of active species in biological systems has historically been limited to transporting said active species into the biological system. Traditionally, the options to change the activity or state of a species already in a biological system without interference from the native biochemical processes has been limited.

Active species, such as drugs typically have their therapeutic action at specific sites in a biological system, such as a body, but are often administered systemically. This typically means that only a small portion of the drug ends up where it is needed, and the rest may cause side effects elsewhere in the body. By delivering drugs locally in an active state, where and when they are needed, a much lower dose can be used, and hence side effects may be avoided. Indeed, many drugs that today fail in clinical trials because of their adverse effects due to high dosages could in fact be effective and without side effects if they were delivered locally and at very low doses. Similarly, improving control of drugs in cell cultures and tissue cultures may allow for more efficient in vitro studies, such as studies in preclinical drug development.

A drug delivery device, such as an implantable device, could provide active drugs locally and provide several benefits. For example, the side effects due to high dosage that is associated with systemic administration of drugs could be avoided. Depending on the application, it may be essential that the drugs can be delivered quickly when needed. This is specifically important when interacting with the nervous system.

Furthermore, it is essential that any implantable device technology have a long lifetime. This means that the device should be stable inside the body, not produce inflammation, and that enough drugs/therapeutic/signalling substances can be stored in the device. The latter requirement typically implies that a reservoir needs to be coupled to the device.

Depending on the application it may be of interest to control the amount of an active drug that is provided at several sites in the biological system (each site can be addressed independently from the others).

There are several methods for local drug delivery already in use, including implanted pumps where the delivery rate can be controlled in time. When the drug is dissolved and delivered in a carrier fluid this dilutes the environment where the drug is delivered, and can lead to an increased pressure if the drug is delivered into a confined compartment. Microfluidics is the scaled down version of drug delivery in fluids, mostly used for in vitro lab-on-a-chip applications. Even though the volumes are much smaller, the same problem with increased pressure still exists. Furthermore, the amount of delivered drug is not controlled to a very high extent for either of these fluidic techniques. A further drawback is that additional mechanical pumps are required, which may be prone to failure. Other techniques used in practice are transdermal patches and subdermal implants that exhibit passive delivery, meaning that drugs are continuously provided at a site at a predetermined rate. The delivery rate can thus not be actively controlled in time with a sufficiently high degree of precision and there is further no spatiotemporal control.

A few techniques for local drug delivery utilize the fact that many drugs and neurotransmitters are, or can occur in, electrically charged form. This implies that they can be controlled and measured electrically. These techniques include drug release from conducting polymers and iontophoresis. Iontophoresis, or electromotive drug administration (EMDA), is a method for administering charged drugs through the skin with an applied electric field. This method is not very precise in terms of the amount of delivered drugs. Charged drugs have also been incorporated as counter ions into conducting polymers, and when the charge of the polymer is altered as a function of oxidation or reduction, the drug (counter ions) is expelled and released from the conducting polymer without any liquid flow. Although many research groups have successfully used this principle, it suffers from high passive leakage, since ions of the electrolyte/body fluid are passively exchanged with the ionic drugs loaded in the conducting polymer, regardless of the addressing voltage. Furthermore, only the drugs originally incorporated into the conducting polymer during the fabrication or pre-usage phase can be released, which limits the amount of drug that can be delivered. Additionally, many drugs cannot be permanently charged, thus appear to be effectively neutral and, hence, cannot be delivered using this technique.

There is thus a need for a system where it is possible to individually control release of both electrically charged and electrically neutral chemical species at one or several sites into a target electrolyte. The following documents describe different aspects in relation to the present teaching:
(1) Daniel T Simon ET AL: "Precise Neurotransmitter-Mediated Communication with Neurons In Vitro and In Vivo Using Organic Electronics", Journal of Biomechanical Science and Engineering, 1 January 2010 (2010-01-01), pages 208-217;
(2) WO 2017/044983 A1 (SHASQI INC [US]) 16 March 2017 (2017-03-16);
(3) US 2019/111251 A1 (BERGGREN MAGNUS [SE] ET AL) 18 April 2019 (2019-04-18).

### SUMMARY OF THE PRESENT DISCLOSURE

One object of the description is to provide a system and method with improved control of the species released and the types of species released.

According to a first aspect there is provided a system for releasing a species. The system comprises a device comprising a body, an ion conductive member, a first electrode, and a second electrode. The system further comprises a source solution, a target solution, a first species, a second species, and an electrical power source connected to the first and second electrode.

A first end of the ion conductive member is arranged in contact with the source solution. A second end of the ion conductive member is arranged in contact with the target solution. The first electrode is arranged in contact with the source solution. The second electrode is arranged in contact with the target solution. The first species is in the source solution, and the second species is in the target solution.

The ion conductive member is configured to, under the influence of an electrical field provided by the electrical power source, allow transport of the first species through the ion conductive member from the source solution to the target solution.

The first species is arranged to interact with the second species in the target solution, such that at least one part of the second species is released.

This has the advantage of allowing the transport of a charged first species into the target solution to govern the release of at least one part of the second species. Further the activity state of a second species may be changed by transporting the first species into the target solution for interaction with the second species. The released at least one part of the second species may comprise a biologically active agent, such as a drug, receptor or acceptor. The released part may enable biological activity, fluorescence activity or deactivate biological activity.

This provide improved control of the species released (positive, negative or neutral) and the types of species released, wherein release may be performed into in vitro and/or in vivo biological systems.

In this disclosure the term "species in a solution" is to be understood in the broadest reasonable interpretation, including any arrangement allowing the species to be accessed by another species which is suspended in the solution. A species immobilized on a surface, wherein the surface is in contact with a solution, is considered in the solution.

In this disclosure the term solution is to be understood in its broadest reasonable interpretations. In one embodiment the first species may be transported into the target solution being in/constituting a volume comprising liquid flowing through said solution. In one embodiment the first species may be transported into the target solution being in/constituting a volume comprised in an extensive fluidic system. In one embodiment the first species may be transported into the target solution being in/constituted by a biological system, such as a living organism, or organs, nerves, joints, cell culture, or a tissue or a bloodstream of an animal or a human. The released at least one part of the second species may be released into a biological system. The spatial limitation of the target solution volume may be dependent on the application and the area which contacts the system. For instance, if the first species is transported into a target solution comprising hydrogel, the volume exposed to the first species may be limited to the extent of the hydrogel. If the system is inserted into a fluid stream, the target solution may be all the fluid that flows past the system. The target solution may, hence, be an *in vivo* solution. In such case the system may for example be used to deliver a biologically active agent to a site in the body. The system may then be arranged to interface with a biological system, such as implantation into a living organism. Alternatively, the system may be used *in vitro* in applications such as controlling the exposure of biologically active agents of cell or tissue cultures

The second species may be immobilized on a surface, wherein the surface is arranged to be in contact with the target solution. The surface may be a surface arranged at the second end of the ion conductive member in the target solution. The second species may be immobilized to/on a particle and/or a 3D scaffold suspended in the target solution or arranged at the second end of the ion conductive member. That the surface is arranged at the second end of the ion conductive member is here meant that the surface may be arranged directly at the interface between the ion conductive member and the target solution or that the surface is arranged at a distance from the ion conductive member.

The second species may alternatively be suspended in the target solution. Thereby the second species may continuously reach the first species entering the target solution via the second end of the ion conductive member, thereby avoiding depletion that may occur for a finite amount of second species immobilized on a surface.

In this disclosure the term release may relate to releasing at least one part of the second species which is suspended in the target solution, or releasing at least on part of the second species wherein said second species is immobilized to a surface, a particle and/or a 3D scaffold such that said part of the second species is free to move independently. The term release may relate to at least one part of the second species separating from the second part of the second species, whereby said at least one part of the second species is free to move. The term release may relate to at least one part of the second species breaking all covalent bonds with the second part of the second species.

The device may comprise a first vessel, wherein the source solution is arranged in the first vessel.

The device may comprise a second vessel, wherein the target solution is arranged in the second vessel.

A vessel may be arranged to accommodate an enclosed solution, accommodate a solution exposed to the environment, or accommodate a part of a solution wherein said solution extends into the environment beyond the vessel. Thereby the source and/or target solution may be in a controlled environment, thereby increasing the freedom to choose the first species and the second species of the system.

The second species may be immobilized on the surface of the second vessel. In one example, the second species is immobilized to the surface of the vessel at the second end of the ion conductive member. That the second species is immobilized at the second end of the ion conductive member is here meant that the second species may be arranged directly at the interface between the ion conductive member and the target solution or that the second species is arranged at a distance from the ion conductive member. Alternatively, the second species is free in solution in the target solution in the second vessel.

The interaction between the first species and the second species may be a bioorthogonal chemical reaction.

The term bioorthogonal chemistry refers to any chemical reaction that is compatible with biomolecules and that can occur inside of living systems without interfering with native biochemical processes. Defined as a highly selective reaction that can occur/proceed in complex reaction environments and/or in the presence of many other naturally occurring functional groups. The bioorthogonal chemistry interactions may be utilized both *in vitro* and *in vivo.* Bioorthogonal chemical reactions comprise bioorthogonal addition reactions and bioorthogonal cleavage reactions.

The interaction between the first species and the second species may comprise a bioorthogonal addition reaction and/or a bioorthogonal cleavage reaction.

Utilizing a bioorthogonal chemical reaction, the system may operate with the target solution, or the source solution, comprising complex biomolecules and species with large number of naturally occurring functional groups.

The first species may be arranged to interact with the second species by at least one elimination reaction, thereby releasing at least one part of the second species into the target solution.

The second species may be synthesized such that at least one part is to be released and the functional group(s) arranged to interact with the first species may be selected independently.

This has the advantage of allowing the second species to be tailored to release a wide selection of biologically active agents upon interacting with the same or similar first species.

The first species may comprises at least one of: azide, phosphine, palladium compound, tetrazine, trans-cyclooctene, cycloalkyne, or derivatives of any one thereof.

The second species may comprise at least one of: tetrazine, trans-cyclooctene, isonitrile, 4-azidobenzyl carbamate, propargyl carbamate, 1,2,3,4-tetrazine-3-carboxamide, mesoionic sydnones, sulfonyl sydnonimines, dibenzoazacyclooctyne (DIBAC), vinyl ether, benzonorbornadiene, or derivatives of any one thereof.

The ion conductive member may be a cation exchange membrane.

Alternatively, the ion conductive member may be an anion exchange membrane.

Using an anion exchange membrane or a cation exchange membrane an improved selectivity in the transport of the first species from the source to target solution can be achieved.

The system may comprise two or more target solutions, wherein the ion conducting member comprises two or more second ends, each second end arranged in contact with a respective target solution, and wherein the ion conductive member is arranged to transport the first species from the source solution to each target solution.

Thereby allowing controlled transport of the first species to each target solution. This may further have the advantage of allowing systems comprising two types of second species, each arranged in a respective target solution. Thereby, interaction of the first species with the two different types of second species may result in the release of different types of released parts in the respective target solution.

The system may comprise two or more source solutions, wherein the ion conducting member comprises two or more first ends, each first end arranged in contact with a respective source solution, and wherein the ion conductive member is arranged to transport the first species from each source solution to the target solution.

Thereby allowing the first species to be transported to the target solution from multiple source solutions. This further has the advantage of allowing the system comprising at least two ion conductive members to be arranged more freely, whereby the transport distance of the first species through the ion conductive member may be reduced.

This further has the advantage of allowing the ion conductive member to transport the first species from the desired source solution to the desired target solution. The further has the advantage of allowing the system comprising at least two types of first species and at least two types of second species to control in which source solution a specific type of second species releases a part.

According to a second aspect there is provided a method for releasing a species. The method comprises the steps of
providing a first species in a source solution;
providing a second species in a target solution;
providing an ion conductive member, wherein a first end of the ion conductive member is arranged in contact with the source solution and a second end of the ion conductive member is arranged in contact with the target solution; and
transporting the first species into the target solution via the ion conductive member by applying an electric field across the ion conductive member, wherein the first species transported to the target solution is arranged to interact with the second species, such that at least one part of the second species is released.

Thereby the release of at least one part of the second species may be controlled by the transport of the first species into the target solution.

The step of providing a first species may further comprise providing a first vessel and arranging the source solution in said vessel. The step of providing a second species may further comprise providing a second vessel and arranging the target solution in said vessel.

Hence, the source and/or target solution may be in a controlled environment, thereby increasing the freedom to choose the first species and the second species.

According to a third aspect there is provided a device for releasing a species, the device comprises a body, a first vessel, a second vessel, an ion conductive member, a first electrode, and a second electrode. The first vessel is arranged to accommodate a source solution. The second vessel is arrange to accommodate a target solution.

A first end of the ion conductive member connects to the first vessel and is arranged to be in contact with the source solution when accommodated in the first vessel. A second end of the ion conductive member connects to the second vessel and is arranged to be in contact with the target solution when accommodated in the second vessel.

The first electrode is arranged at the first vessel and is arranged to be in contact with the source solution when accommodated in the first vessel. The second electrode is arranged at the second vessel and is arranged to be in contact with the target solution when accommodated in the second vessel. The first vessel is arranged to accommodate a first species in the source solution. The second vessel is arranged to accommodate a second species in the target solution.

The ion conductive member is arranged to, under the influence of an applied electrical field allow transport of the first species through the ion conductive member from the first vessel to the second vessel, wherein the first species transported to the second vessel is arranged to interact with the second species, whereby at least one part of said second species is released.

According to a fourth aspect there is provided a use of the device described above for releasing at least one part of the second species into the target solution by providing the first species in the source solution in the first vessel, providing the second species in the target solution in the second vessel, and applying an electric field between the first and second electrode.

According to a fifth aspect there is provided a method of treating and/or preventing a disease or disorder in an animal or human by controlling the release of a biologically active agent, the method comprising: providing a system or a device, as presently disclosed, configured and dimensioned to be used within a body of an animal or human; interfacing the system or device with the body of the animal or human or arranging the system or device in the body of the animal or human; and applying an electric field across the ion conductive member, wherein the first species is transported to the target solution and interacts with the second species, such that at least one part of the second species is released, wherein the at least one part of the second species comprise the biologically active agent. The present invention is set out in the appended claims.

### BRIEF DESCRIPTION OF THE DRAWINGS

Fig. 1a and 1b depicts schematically a system for releasing a species.
Fig. 2 depicts schematically a multi-path system for releasing a species.
Fig. 3a-g illustrates interactions between first and second species.
Fig. 4 shows schematically a method for releasing a species.
Fig. 5 illustrates an example interaction between a first and a second species.
Fig. 6 illustrates a set of example tetrazin-based first species

### DETAILED DESCRIPTION

Throughout the figures, same reference numerals refer to same parts, concepts, and/or elements. Consequently, what will be said regarding a reference numeral in one figure applies equally well to the same reference numeral in other figures unless not explicitly stated otherwise.

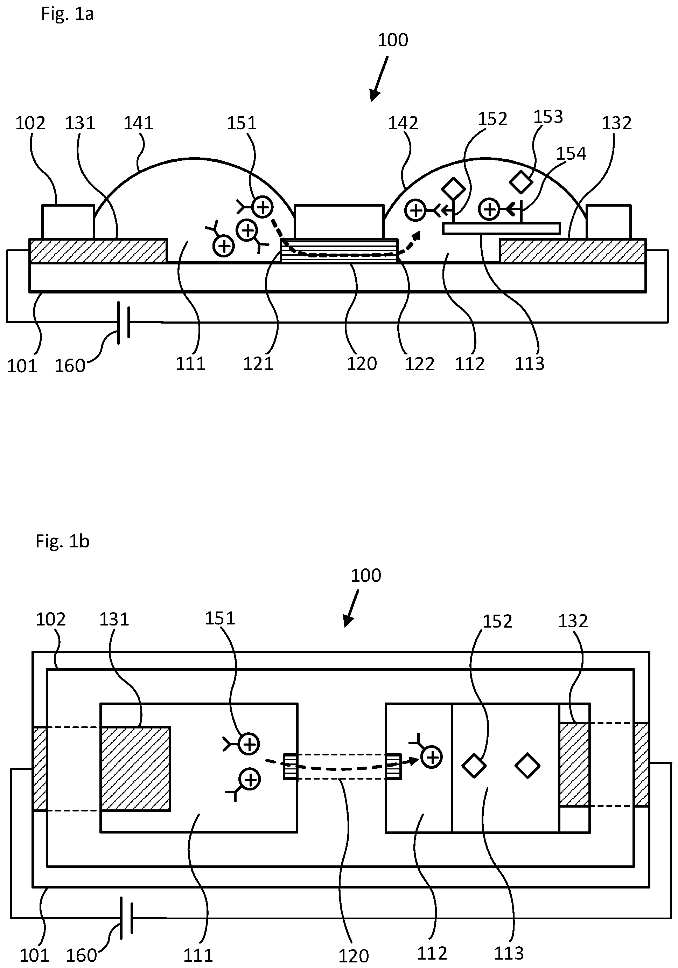

Fig. 1a and 1b depicts schematically a system for releasing a species. The example system 100 is arranged to transport a first species 151 from a source solution 141 to a target solution 142, whereby the transported first species 151 is arranged to interact with a second species 152 in the target solution 142 and release at least one part 153 of the second species 152. Fig. 1a and 1b include representations of the first species 151 and a path of transport; the second species 152 immobilized on a surface 113; and an example interaction between the first 151 and the second species 152 during use of the system 100. The species representation is not to scale and individual species are typically not visible to the human eye.

Fig. 1a shows schematically a cross-sectional side view of the example system 100 for releasing a species. The system 100 comprises a device 101, wherein the device comprises a body 102, an ion conductive member 120, a first electrode 131 and a second electrode 132. The system 100 further comprises the source solution 141, the target solution 142, the first species 151, the second species 152, and an electrical power source 160 connected to the first electrode 131 and the second electrode 132.

This example system 100 may further comprise a first vessel 111, wherein the first vessel 111 accommodates the source solution 141. This example system 100 may further comprise a second vessel 112, wherein the second vessel 112 accommodates the target solution 142.

A first end 121 of the ion conductive member 120 is arranged to be in contact with the source solution 141. A second end 122 of the ion conductive member 120 is arranged to be in contact with the target solution 142. The first electrode 131 is arranged to be in contact with the source solution 141. The second electrode 132 is arranged to be in contact with the target solution 142.

In this example the system 100 the first end 121 of the ion conductive member 120 is connected to the first vessel 111; the second end 122 of the ion conductive member 120 is connected to the second vessel 112; the first electrode 131 is arranged at the first vessel 111; and the second electrode 132 is arranged at the second vessel 112.

The first species 151 is in the source solution 141. In this example system 100 the first species 151 is a cation suspended in the source solution 141 comprised in the first vessel 111. In another example the first species 151 may be an anion suspended in the source solution 141 comprised in the first vessel 111.

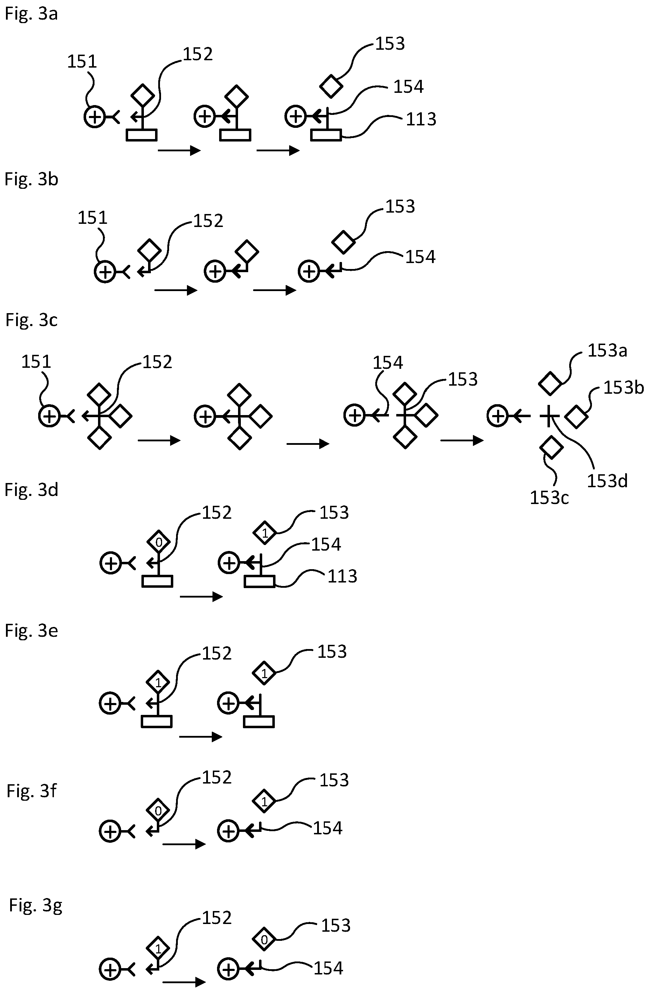

The first species 151 may be suspended in the source solution 141. The second species 152 is in the target solution 142. In this example the second species 152 is immobilized on a surface 113 comprised in the second vessel 112. Alternatively, as illustrated in Fig. 3b, the second species 152 may by suspended in the target solution 142. With immobilized on a surface is meant attaching a species, such as covalently bonding, to said surface whereby the species movement is restricted. In one example the second species 152 comprising a thiol group covalently bonded to a gold surface 113.

The second species 152 may be immobilized on at least one surface, particle and/or 3D scaffold arranged in contact with the target solution 142.

The second species 152 may be immobilized on the walls of the second vessel 112.

The second species 152 may be immobilized at the second end 122 of the ion conductive member 120. The second species 152 may be immobilized on the second end 122 of the ion conductive member 120.

The ion conductive member 120 is configured to, under the influence of an electrical field provided by the electrical power source 160, allow transport of the first species 151 through the ion conductive member 120 from the source solution 141 to the target solution 142. In this example system 100, applying an electric field towards the second vessel 112 transports the positively charged first species 151 to the second vessel 112 via the ion conductive member 120.

The first species 151 is arranged to interact with the second species 152 in the target solution 142, whereby at least one part 153 of the second species 152 is released. In this example system 100 the first species 151 is arranged to interact with the second species 152 immobilized on the surface 113, whereby a first part 153 of the second species 152 may be released into the target solution 142 and the first species 151 is bound to a remaining second part 154 of the second species 152 immobilized on the surface 113.

The released first part 153 of the second species 152 may comprise a biologically active agent, such as a drug, a reporter or an effector.

In another non-illustrated example the second species 152 may be immobilized on the surface 113 via a first part 153 of the second species 152, whereby the first species 151 interacting with the second species 152 may cause a second part 154 of the second species 152 bound to the first species 151 to be released into the target solution 142.

In another example at least one first part 153 of the second species 152 is arranged to functions as a first species 151 upon release, whereby said released at least one first part 153 is arranged to interact with another unreacted second species 152. In one example, the second species 152 is configured to release two first parts 153 each functioning as a first species 151 upon release, whereby an initial interaction between the first species 151 and the second species 152 may trigger a chain reaction of interactions.

In another non-illustrated example, the first species 151 may interact with an immobilized second species 152, whereby the whole second species 152 is released into the target solution 142. Thereby a clean, non-functionalized surface is produced. Such a system could be used if wanting to analyse for example the amount of released compound. If the surface is for example a quartz crystal microbalance (QCMD) or a surface plasmon resonance (SPR) active element, the release could be monitored and checked. Alternatively, impedance spectroscopy could be used.

The first 121 and second end 122 of the ion conductive element 120 may comprise a transport interface between the ion conductive element 120 and the corresponding source solution 141 and target solution 142.

During use of the system 100, the electrodes 131,132, the solutions 141,142 and the ion conductive member 120 may be configured to, upon applying an electric field between the electrodes 131,132, allow a current to run through the system 100 between the electrodes 131,132.

The power source 160 may be arranged to maintain a constant current through the system 100. The power source 160 may be arranged to maintain a constant voltage between the first 131 and second electrode 132.

The power source 160 may be arrange to provide potential between and/or current through the first 131 electrode and the second electrode 132 according to a predetermined program.

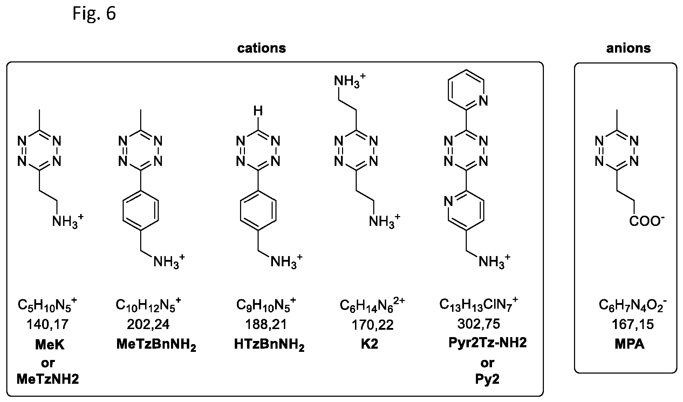

The ion conductive member 120 may be configured to, under the influence of an electrical field provided by the electrical power source 160, allow transport of at least two types of first species 151 from the source solution 141 to the target solution 142 via the ion conductive member 120. In one example the ion conductive member is configured to allow transport of two tetrazine (Tz) based species, MeTzNH₂ and MeTzBnNH₂ seen in Fig. 6, dissolved in an aqueous source solution 141 at physiological pH to the target solution 142 via the ion conductive member 120.

The ion conductive member 120 may be configured to, under the influence of an electrical field provided by the electrical power source 160, allow transport of at least one additional type of first species 151 (not shown) through the ion conductive member 120 from the target solution 142 to the source solution 141.

The rate at which the first species 151 is transported through the ion conductive member 120 may be approximately proportional to the voltage applied between the electrodes 131,132. At least one electrode 131,132 may be an electrochemically active electrode, such as a PEDOT:PSS electrode. An inherent advantage of a system 100 which utilizes electrochemically active polymer electrodes 131,132 is the low voltage required to effect transport of the first species 151 from the source solution 141 to the target solution 142.

The magnitude and polarity of the voltages to be applied across the system may vary depending on a number of factors, such as choice of electrode material(s), the first species 151 to be transported, the distance over which the first species 151 is transported. The polarity of the applied voltages will easily be selected by a person skilled in the art, taking into account the type of charge (positive or negative) of the first species 151 to be transported. The magnitude of the voltage to be applied may in the light of the present invention easily be determined in order to transport a desired amount of the first species 151.

The voltage applied across the ion conductive member 120 may for example be within the range of from about 0.01 V to about 100 V. The optimal voltage to be applied between the electrodes 131,132 will depend on the characteristics of the electrode material used, the solutions 141,142 used, the first species 151 to be transported and the manner in which the voltage is applied. The applied voltage may be AC, DC and/or pulsed. However, the voltage is preferably in the range of from 0.01 V to 100 V, more preferably in the range of from 0.01 V to 20 V.

The first electrode 131 and/or the second electrode 132 may be arranged in direct and/or indirect contact with the respective source solution 141 and target solution 142.

The surface area of the electrodes 131,132 may be in the range of 1 cm² to 25 cm². The surface area of the electrodes 131,132 may be 100 µm² to 1 cm² or 25 cm² to 1 dm².

The system 100 may comprise at least one additional spatially separated ion conductive member 120 (not illustrated). Each of the at least two spatially separated ion conductive members 120 may be arranged to allow transport of the first species 151.

Thereby an array of different types of ion conductive members may be utilized in the same system. An increased number of types of the first species may then be utilized simultaneously in the same system.

The ion conductive member 120 may be a cation exchange membrane. The ion conductive member 120 may be an anion exchange membrane. The system 100 comprising two or more ion conductive members 120 may comprise a cation exchange membrane and an anion exchange membrane.

The ion conductive member 120 may comprise at least two branches, e.g. be tree like, wherein each branch end 122 is in contact with the target solution 142. The at least two branches may share a common point of intersection within the ion conductive member 120. In one example the ion conductive member 120 comprising two branches forms a "Y"-shaped ion conductive member 120.

The ion conductive member 120 may be designed and/or treated in such a way as to minimize/reduce electric conductivity, but preserve or provide ionic conductivity.

The ion conductive member 120 may comprise a solid and/or a semi-solid material.

The ion conductive member 120 may consist of one material, such as overoxidized poly(3,4-ethylenedioxythiophene) polystyrene sulfonate (PEDOT:PSS).

The ion conductive member 120 may be arranged to transport the first species through the ion conductive member 120 while restricting convective transport of the source solution 141 through the ion conductive member 120 into the target solution 142.

In one embodiment, the ion conductive member 120 comprises a polymer material arranged to allow transport of the first species 151 upon application of an electric field, and restricting convective transport of the source solution 141 through the ion conductive member 120. The ion conductive member 120 may comprise at least one gel, hydrogel, polymer brush, and/or metal organic framework. In one example the ion conductive member 120 comprises a hydrogel comprising a network of polymer chains obstructing any open path for macroscopic bulk flow of solution through the ion conductive member 120.

In this disclosure the terms "restrict convective transport" is to be understood as not providing any unobstructed fluid path for pressure driven flow with a diameter above 1 µm. For example the term would exclude the use of an open capillary channel with a diameter of 10 µm as ion conductive members 120.

The ion conductive member 120 may be arranged to restrict convective transport of the target solution 142 through the ion conductive member 120.

The ion conductive member 120 and the first species 151 may be configured to achieve high convectional and diffusional hindrance of the ion conductive member 120 for the first species 151.

In some embodiments, the ion conductive member 120 may for example have a cross sectional area in the range of 10 nm²-1000 mm², such as 10 mm²-100 mm², such as 10 nm²-10 000 µm², such as 10 nm²-100 µm², such as 10 nm²-1 µm², such as 10 nm²-10 000 nm², or such as 10 nm²-100 nm². In other embodiments the ion conductive member 120 may have a cross sectional area which is smaller than 10 nm².

The ion conductive member 120 may be within the range of 5 µm to 0.3 m in length. The first 121 and second end 122 of the ion conductive member 120 may contact the source solution 141 and/or target solution 142 across at least 1 mm².

The ion conductive member 120 may comprise means for limiting the electronic current between the first 131 and second electrode 132 upon applying an electric field between the electrodes 131,132.

The ion conductive member 120 may comprise a first pre-load region comprising the first species 151. The ion conductive member 120 may comprise at least two pre-load regions, wherein each pre-load region comprises the first species 151. The term pre-load region relates to a region comprising a desired species prior to operating the system.

A faster response, i.e. a faster delivery of the first species 151 from the ion conducting member 120 to the target solution 142, may be achieved by the pre-loading of first species 151 into a region of the ion conductive member 120, since said region may be located at a short distance from the second end 122 of the ion conducting member 120.

The ion conductive member 120 may be formed of a wire, or tube or fibre, which may be coated with e.g. an electrochemically active material such as PEDOT:PSS. The wire, tube or fibre may hence be a supporting substrate, such as a plastic rod or tube. The ion conductive member 120 may further be overoxidized or insulated with an insulation material, such as a photoresist or a silicone glue.

The ion conductive member 120 may, according to one, not shown, embodiment, have a circular cross-section of varying dimensions, such that a portion of the ion conductive member 120 may have an increased cross-section. This increased cross-sectional portion may allow for the first species 151 to be pre-loaded into the ion conductive member 120. The portion having an increased cross- section may be designed specifically to be able to retain a certain amount of the first species 151. The ion conductive member 120 may be provided with any number of such increased cross-sectional portions, i.e. any number of pre-loading areas. The pre-loading areas may also be realized in an ion conductive member 120 having a cross-section which is rectangular or square, or of any other suitable geometry. The ion conductive member 120 may also have a conical shape, i.e. be tapered. The ion conductive member 120 may be formed as a plug arranged at the second vessel 112.

The system 100 may comprise a unitary section comprising the ion conductive member 120 and the first electrode 131.

The source solution 141 and/or the target solution 142 may be an electrolyte solution. The source solution 141 and/or the target solution 142 may be an aqueous solution. The source solution 141 may comprise aqueous pH buffer solution, and the first species 151 may be suspended in the source solution 141.

The source solution 141 and/or target solution 142 may comprise at least one gel, hydrogel, polymer, and/or metal organic framework.

The source solution 141 and target solution 142 used may be based on a solvent that permits ionic conduction in the solution 141,142, i.e. that allows for the dissociation of ionic substances such as salts, acids, bases, etc. Possible solutions 141,142 for use in combination with the present invention may be solutions of salts, acids, bases, or other ion-releasing agents in solvents that support the dissociation of ionic species, thus allowing ionic conductivity. In applications where it is required, the source solution 141 and/or target solution 142 may comprise buffer solutions, such as buffer solutions suitable for use with living organisms or biomolecules, such as proteins. Examples of such buffers include phosphate-buffered saline (e.g. Na2HPO4, KH2PO4, NaCl and KCl) and sodium acetate. As other non-limiting examples of possible solutions, mention can be made of: aqueous solutions of potassium acetate, calcium acetate, NaCl, Na2SO4, H3PO4, H2SO4, KCI, RbNO3, NH4OH, CsOH, NaOH, KOH, H2O2; organic solvents such as acetonitrile, pyridine, DMSO, DMF, dichloromethane, etc., in combination with suitable salts, such as lithium perchlorate and tertiary ammonium salts, e.g. tetra-butyl ammonium chloride; inorganic solvents such as hypercritical CO2, liquid SO2, liquid NH3, etc., in combination with salts that dissociate in these solvents; solvents displaying auto-dissociation, which results in the formation of ionic species, such as water, formic acid and acetic acid.

The source solution 141 and/or target solution 142 may comprise cell culturing media or ingredients thereof, such as proteins, amino acids, vitamins and growth factors.

The source solution 141 and/or target solution 142 may also be in a semi-solid or solidified form, preferably comprising an aqueous or organic solvent-containing gel as described above, such as PEG (polyethylene glycol) hydrogels. However, solid polymeric electrolyte solutions are also contemplated and fall within the scope of the present invention. Furthermore, the term solution encompasses liquid electrolyte solutions soaked into, or in any other way hosted by, an appropriate matrix material, such as a paper, a fabric or a porous polymer.

The target solution 142 may comprise tissue and/or body fluids and/or cells. The target solution 142 may comprise *in vitro* and/or *in vivo* human cells.

The first species 151 may be a cation. The ion conductive member 120 may be a cation conductive member. The ion conductive member 120 may be a cation selective membrane.

The first species 151 may be anion. The ion conductive member 120 may be an anion conductive member. The ion conductive member 120 may be an anion selective membrane.

The first species (151;251) may comprise at least one of:
azide, phosphine, palladium compound, tetrazine, trans-cyclooctene, cycloalkyne, or derivatives of any one thereof.

The second species (152) may comprise at least one of: tetrazine, trans-cyclooctene, isonitrile, 4-azidobenzyl carbamate, propargyl carbamate, 1,2,3,4-tetrazine-3-carboxamide, mesoionic sydnones, sulfonyl sydnonimines, dibenzoazacyclooctyne (DIBAC), vinyl ether, benzonorbornadiene, or derivatives of any one thereof.

In one example the first species 151 may comprise tetrazine (Tz) and the second species 152 may comprise trans-cyclooctene (TCO).

The system 100 may comprise at least two types of first species 151.

The system 100 may comprise at least two types of second species 152.

In this disclosure the term "two types of first species" is to be understood as two distinct species, wherein each type of first species 151 is arranged to interact with a second species 152 such that at least one part 153 of said second species 152 is released. The term "two types of second species" is to be understood as two distinct species, wherein each type of second species 152 is arranged to interact with a first species 151 such that at least one part 153 of said second species 152 is released.

The interaction between the first species 151 and the second species 152 may comprise at least one elimination reaction. An elimination reaction is a type of reaction in which two substituents are removed from a molecule in either a one or two-step mechanism.

The interaction between the first species 151 and the second species 152 may comprise at least one click chemistry interaction, *i.e.* a bioorthogonal cleavage reaction. Click reactions allow the specific joining of a first molecule with a second molecule, characterized by a high thermodynamic driving force that drives the reaction quickly and irreversibly.

The interaction between the first species 151 and the second species 152 may comprise at least one bioorthogonal chemical interaction.

The term bioorthogonal chemistry refers to any chemical reaction that is compatible with biomolecules and that can occur inside of living systems or in *in vitro* systems without interfering with native biochemical processes. Defined as a highly selective reaction that can occur/proceed in complex reaction environments and/or in the presence of many other naturally occurring functional groups. Bioorthogonal chemical reactions comprise bioorthogonal addition reactions and bioorthogonal cleavage reactions.

The interaction between the first species 151 and the second species 152 may comprise a bioorthogonal addition reaction and/or a bioorthogonal cleavage reaction.

The second species 152 may comprise at least one self-immolative linker. A self-immolative linker is a part of a molecule linking at least two parts of said molecule arranged to as a response to an input reaction collapse, whereby at least one linked part is released. In one example the released part 153 of the second species 152 may comprise a self-immolative linker, wherein the reaction releasing the part 153 may be the input reaction for the collapse of the self-immolative linker, whereby the release of the part 153 of the second species 152 may result in multiple released parts 153.

The device 101 may be at least partially covered by a solution containment
structure (not shown), comprising for example polyimide, PDMS, photoresist or any other material which will provide physical, ionic and/or electric insulation of the device 101 or parts thereof, with openings for at least one solution 141,142.

The body 102 of the device 101 may comprise at least one structural element arranged to form the vessels 111,112, and/or partially encapsulate at least one other component of the body 101. In one example the body 102 comprises at least one layer of inert materials, such as photoresist.

The ion conductive member 120 may be partially encapsulated by the body 102 of the device 101. The ends 121,122 of the ion conductive member 120 may be defined by the parts not encapsulated by the body 102.

The body 102 and ion conductive member 120 may be flexible. In one example the body 102 and ion conductive member 120 comprise flexible polymer materials.

The source solution 141 may be enclosed. In one example the first vessel 111 is a closed container, and the second vessel 112 is open to the environment. This allowing the source solution 141 to be in a controlled environment, and allows the target solution 142 to be in contact with the environment. This allows the system 100 to be designed more freely as changes in the environment would be expected to mainly impact the target solution 142, second electrode 132 and/or second species 152.

Fig. 1b shows schematically a top view of the example system 100 for releasing a species. The example system 100 may be the system 100 described in Fig. 1a.

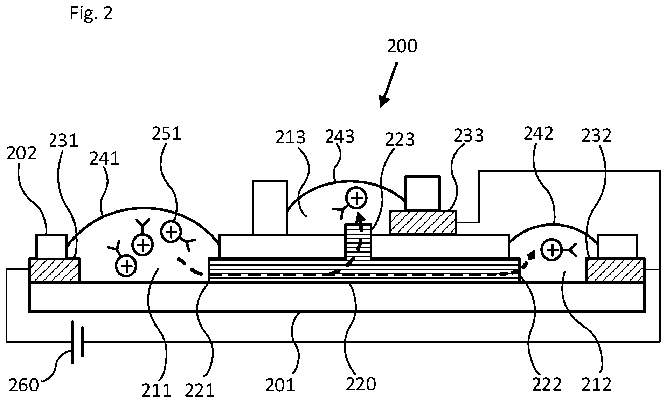

Fig. 2 shows schematically a cross-sectional side view of an example system 200 for releasing a species comprising two target solutions 242,243. Fig. 2 includes a representation of the first species 251 and two paths of transport during use of the system 200.

The system 200 comprises a device 201, wherein the device comprises a body 202, an ion conductive member 220, a first electrode 231, a second electrode 232 and a third electrode 233. The system 200 further comprises a source solution 241, a first target solution 242, a second target solution 243 and an electrical power source 260 connected to the first 231, the second 232 and the third electrode 233. The system further comprises a first species 251 and a second species (not shown).

This example system 200 may further comprise a first vessel 211, a second vessel 212, a third vessel 213, wherein the first vessel 211 accommodates the source solution 241; the second vessel 212 accommodates the first target solution 242; and the third vessel 213 accommodates the second target solution 243.

The first target solution 242 and the second target solution 243 may be in direct and/or indirect contact. The first target solution 242 and the second target solution 243 may be the same solution.

A first end 221 of the ion conductive member 220 may be arranged to be in contact with the source solution 241. A second end 222 of the ion conductive member 220 may be arranged to be in contact with the first target solution 242. A third end 223 of the ion conductive member 220 may be arranged to be in contact with the second target solution 243.

In this example system 200 the first end 221 of the ion conductive member 220 is connected to the first vessel 211; the second end 222 of the ion conductive member 220 is connected to the second vessel 212; and the third end 223 of the ion conductive member 220 is connected to the third vessel 213. In this example one ion conductive member 120 is connected to the first 211, second 212 and third vessel 213. In another example the ion conductive member 120 may be connected to the first 211 and second vessel 212, and an additional spatially separate ion conductive member 120 may be connected to the first 211 and third vessel 213.

The first electrode 231 may be arranged to be in contact with the source solution 241. The second electrode 232 is arranged to be in contact with the first target solution 242. The third electrode 233 is arranged to be in contact with the second target solution 243.

In this example system 200 the first electrode 231 is arranged at the first vessel 211; the second electrode 232 is arranged at the second vessel 212; and the third electrode 233 is arranged at the third vessel 213.

The first species 251 is in the source solution 241. In this example system 200 the first species 251 is a cation suspended in the source solution 241 comprised in the first vessel 211. In another example the first species 251 is an anion suspended in the source solution 241 comprised in the first vessel 211.

The second species is in the first target solution 242 and in the second target solution 243.

The ion conductive member 220 is configured to, under the influence of an electrical field provided by the electrical power source 260, allow transport of the first species 251 through the ion conductive member 220 from the source solution 241 to the first target solution 242 and second target solution 243. In this example applying an electric field from the first vessel 211 towards the second vessel 212 and third vessel 213 transports the positively charged first species 251 to the second vessel 212 and third vessel 213 via the ion conductive member 220.

In this example the system 100 shows that the same potential is applied to the second electrode 232 and the third electrode 233 by the power source 260. The system 200 may be configured to independently set a potential between the first 231 and second electrode 232, and between the first 231 and third electrode 233.

The first species 251 is arranged to interact with the second species in the first target solution 242 and/or the second target solution 243, whereby at least one part of the second species is released.

The system 200 may comprise a plurality of vessels such as at least three, at least four, at least five, at least six vessels or n vessels 211,212,213.

The system 200 may comprise at least three target solutions 242,243, wherein each target solution 242,243 is in contact with the respective end 222,223 of the ion conductive member 220, and wherein the ion conductive member 220 is arranged to allow transport of the first species 251 from the source solution 241 to the at least three target solutions 242,243.

The system 200 may comprise at least two source solutions 241, wherein each source solution 241 is in contact with the respective end 221 of the ion conductive member 220, and wherein the ion conductive member 220 is arranged to allow transport of the first species 251 from each source solution 241 to at least one target solution 242.

The system 200 may comprise at least two source solutions 241, and at least two target solutions 242,243.

The system 200 may comprise at least two types of first species 251. The system 200 may comprise at least two types of second species.

At least one type of first species 251 may be in each source solution 241. At least one type of second species may be in each target solution 241. Each first species 251 may be arranged to release at least one part of at least one second species upon interacting with said second species.

The ion conductive member 220 may be arranged to for each source solution 241 allow at least one type of first species 251 to be transported from said source solution 241 to at least one target solution 242,243.

At least one end 221,222,223 of the ion conductive member 120 may comprise a part (not shown) arranged to regulate the transport of the first species 251. The part arranged to regulate the transport of the first species 251 may be controlled by an additionally applied electric field. The part arranged to regulate the transport of the first species 251 may comprise an ion-diode arranged to regulate ion transport rates based on applied electric field.

Fig. 3a-3g schematically illustrate the release of at least one species. Fig. 3a-3g show a first species 151 interacting with a second species 152 whereby at least one part 153 of the second species 152 is released. In the examples shown with the second species immobilized on a surface 113 the first species 151 remains bound to a second part 154 of the second species 152 that remains immobilized. The first species 151, the second species 152 and the immobilization of the second species 152 may be configured to allow the first species 151 to bind to the part 153 of the second species 152 releasing from the surface 113.

Fig. 3a shows leftmost the first species 151 free to interact with the second species 152 immobilized on a surface 113. The centre shows an intermediate step of the first species 151 binding to the second species 152. Rightmost the release of the first part 153 of the second species 152 from the surface is shown. In this example the first species 151 remains bound to the second part 154 of the second species 152. In another example the first species 151 is released from the second part 154 of the second species 152. In another example the first species 151 interacts with the immobilized second species 152, whereby the second species 152 is released from the surface, thus resulting in an unfunctionalized surface.

Fig. 3b shows leftmost the first species 151 free to interact with the second species 152 suspended in solution. The centre shows an intermediate step of the first species 151 binding to the second species 152. Rightmost shows the release of the first part 153 of the second species 152 from second part 154 of the second species 152 and the bound first species 151. In this example the first species 151 remains bound to the second part 154 of the second species 152. In another example the first species 151 is released from the second part 154 of the second species 152.

Fig. 3c shows leftmost the first species 151 free to interact with the second species 152 suspended in solution. The next illustration shows an intermediate step of the first species 151 binding to the second species 152. The next illustration shows the release of the first part 153 of the second species 152 from second part 154 of the second species 152 and the bound first species 151. Rightmost the first part 153 of the second species 152 breaks into four parts 153a,b,c,d. In one example the first part 153 of the second species 152 comprises a self-immolative linker 153d arranged to cleave, whereby at least one part 153a,b,c of the first part 153 of the second species 152 is released. In this example the second species 152 is shown as suspended in solution. In another example the second species 152 is immobilized. In one example at least one first part 153 of the second species 152 is arranged to functions as a first species 151 upon release, whereby said released at least one first part 153 is arranged to interact with another unreacted second species 152.

The interaction shown in Fig. 3c, where one species results in the release of at least two other species 153a,b,c,d, may be utilized to increase the result of transporting the first species 151 to the second species 152.

Fig. 3d-3g illustrate changes in activity of the part 153 released from the second species 152. A "1" is used to indicate an active state of the part 153, and a "0" is used to indicate an inactive state of the part 153. Fig. 3d-3e relate to the interaction shown in Fig. 3a. Fig. 3f-3g relate to the interaction shown in Fig. 3b. The intermediate step has been omitted.

Fig. 3d shows leftmost the first species 151 free to interact with the second species 152 immobilized on a surface 113, wherein the part 153 of the second species 152 to be released is in the inactive state. Rightmost the release of the first part 153 of the second species 152 from the surface is shown, wherein the first part 153 is in the active state.

Fig. 3e shows leftmost the first species 151 free to interact with the second species 152 immobilized on a surface 113, wherein the part 153 of the second species 152 to be released is in the active state. Rightmost the release of the first part 153 of the second species 152 from the surface is shown, wherein the first part 153 is in the active state.

Fig. 3f shows leftmost the first species 151 free to interact with the second species 152 suspended in solution, wherein the part 153 of the second species 152 to be released is in the inactive state. Rightmost shows the release of the first part 153 of the second species 152 from the second part 154 of the second species 152 and the bound first species 151, wherein the first part 153 is in the active state.

Fig. 3g shows leftmost the first species 151 free to interact with the second species 152 suspended in solution, wherein the part 153 of the second species 152 to be released is in the active state. Rightmost shows the release of the first part 153 of the second species 152 from the second part 154 of the second species 152 and the bound first species 151, wherein the first part 153 is in the inactive state.

In one embodiment an agreement comprises a system 100 as described in Fig. 1 and at least one additional device 101, wherein each second vessel 112 is in contact with a fluid flow, such as a blood flow or flow of cell medium. The first device 101 may be arranged to transport a first type of first species 151 into the corresponding second vessel 112, whereby the first species 151 interacting with the second species 152 results in an active first part 153 of the second species 152 being released into the fluid flow. The second vessel 112 of the second device 101 may be in contact with the fluid flow downstream of the second vessel 112 of the first device 101. The second device 101 may be arranged to transport a second type of first species 151 into the corresponding second vessel 112, whereby the first species 151 interacting with the active first part 153 of the second species 152 transported via the fluid flow results in a deactivated first part 153 of the second species 152.

The use of multiple devices 101 wherein each device 101 is arranged to transport a first species 151 into a fluid flow may be utilized to influence where in the fluid flow path a species, such as a drug, is in an active state.

In another embodiment the systems 200 as described in fig. 2 comprising two target solutions 242,242, wherein each target solutions 242,243 is in contact with a fluid flow. The system 200 may be arranged to transport a first type of first species 251 into the first target solution 242, whereby the first species 251 interacting with the second species results in an active first part of the second species being released into the fluid flow. The second target solution 243 may be in contact with the fluid flow downstream of the first target solution 242. The system 200 may be arranged to transport a second type of first species 251 into the second target solution 243, whereby the first species 251 interacting with the active first part of the second species transported via the fluid flow results in a deactivated first part of the second species.

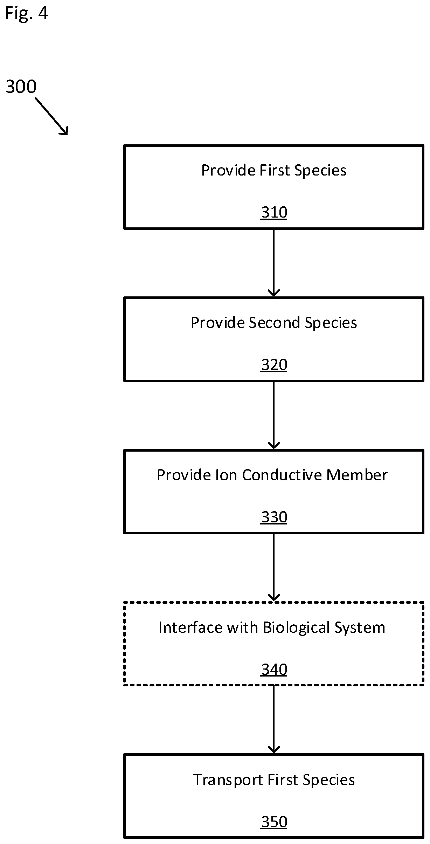

Fig. 4 shows schematically a method for releasing a species.

The method 300 comprises the steps of
providing 310 a first species in a source solution;
providing 320 a second species in a target solution;
providing 330 an ion conductive member, wherein a first end of the ion conductive member is arranged in contact with the source solution, and wherein a second end of the ion conductive member is arranged in contact with the target solution; and
transporting 350 the first species into the target solution via the ion conductive member by applying an electric field across the ion conductive member, wherein
the first species transported to the target solution is arranged to interact with the second species, such that at least one part of the second species is released.

The step of providing 310 a first species may further comprise providing a first vessel and arranging the source solution in said vessel, and/or
the step of providing 310 a second species may further comprise providing a second vessel and arranging the target solution in said vessel.

The method may comprise providing 320 the second species in at least two target solutions, and transporting 350 the first species into the at least two target solutions via the ion conductive member.

The step of providing 330 the ion conductive member may comprise providing at least two spatially separate ion conductive members.

The method may comprise a step of interfacing 340 the target solution with a biological system. In one example the step of interfacing 340 comprises implanting at least the second end of the ion conducive member into a living organism. In one example the step of interfacing 340 comprises arranging the second end of the ion conducive member in an in vitro cell culture.

In one example the method is suitable for treating and/or preventing a disease or disorder in an animal or human by controlling the release of a biologically active agent, the method comprises: providing a system or a device, as presently disclosed, configured and dimensioned to be used within a body of an animal or human; interfacing 340 the system or device with the body of the animal or human or arranging the system or device in the body of the animal; and applying an electric field across the ion conductive member, wherein the first species is transported 350 to the target solution and interacts with the second species, such that at least one part of the second species is released, wherein the at least one part of the second species comprise the biologically active agent.

The target solution of the system described above or used with the device or in the method described above may comprise and/or may be in contact with any one of tissue, body fluid(s) and cells.

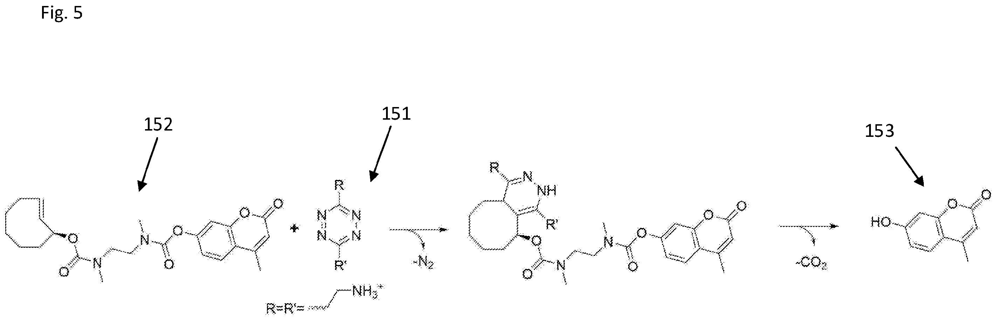

Fig. 5 shows an example interaction between a first and a second species.

In this example the first species 151 is 2,2'-(1,2,4,5-tetrazine-3,6-diyl)bis(ethan-1-amine) hydrochloride, which under physiological conditions forms the positively charged compound 2,2'-(1,2,4,5-tetrazine-3,6-diyl)bis(ethan-1-aminium). The second species 152 is (S,E)-cyclooct-2-en-1-yl (4-methyl-2-oxo-2H-chromen-7-yl) ethane-1,2-diylbis(methylcarbamate).

In this example the first species 151 and second species 152 interacting results in one part 153 releasing, wherein the released part 153 comprises (4-Methylumbelliferone) configured to function as an active fluorophore upon release.

In this example R and R' of the first species 151 are selected to form 2,2'-(1,2,4,5-tetrazine-3,6-diyl)bis(ethan-1-aminium), a similar interaction with the second species 152 of this example may be possible with multiple types of tetrazine-based first species 151 comprising other R and R'.

Fig. 6 shows a set of example tetrazin-based first species. The chemical structure, structure formula, molecular weight and abbreviation is listed for each example first species.

Fig. 6 shows non exhaustive set of first species based on tetrazin which are positively charged under physiological condition:
MeTzNH3+ is 2-(6-methyl-1,2,4,5-tetrazin-3-yl)ethan-1-aminium, the amine precursors (hydrochlorides) form ammonium salts (R-NH3+) under physiological conditions.
MeTzBnNH3+ is (4-(6-methyl-1,2,4,5-tetrazin-3-yl)phenyl)methanaminium.
HTzBnNH3+ is (4-(1,2,4,5-tetrazin-3-yl)phenyl)methanaminium.
K2 is 2,2'-(1,2,4,5-tetrazine-3,6-diyl)bis(ethan-1-aminium).
Pyr2TzNH3+ is (6-(6-(pyridin-2-yl)-1,2,4,5-tetrazin-3-yl)pyridin-3-yl)methanaminium.

Fig. 6 shows an example of a first species based on tetrazin which is negatively charged under physiological condition:
MPA is 3-(6-methyl-1,2,4,5-tetrazin-3-yl)propanoate.

Turning back to Fig. 1a and 1b, and Fig. 5, an example of fabricating and using the system of the present disclosure will now be described. The fabrication and use of the present disclosure is in no way limited by the described scenario example.

In this example the main steps of fabricating a device 101 corresponding to the illustration in Fig. 1a will be described. 50nm gold contacts are added to a clean glass substrate by thermal evaporation and a lift-off process, defining the gold electrodes 131,132. Spin coat a 500 nm layer of poly(4-styrenesulfonic acid-co-maleic acid) (PSS-co-MA) cross-linked with polyethylene glycol (PEG), the material which will become the ion conductive member 120. Deposit photoresist on top of the PSS-co-MA/PEG layer, expose photoresist and develop to form a 1 mm long and 3 mm wide ion conductor member 120. In order to form the barrier separating the two sides, or vessels 111,112, at each end 121,122 of the ion conductive member 120 another application of photoresist is performed, followed by exposure and development. The resulting photoresist structure forms the part of the body 102 of the device 101 separating and/or forming the vessels 111,112, wherein each vessel 111,112 is in contact with a gold electrode 131,132 and an end 121,122 of the ion conductive member 120.

A voltage source is used as the power source 160, and it is connected to the gold electrodes 131,132.

The source solution 141 is an aqueous solution comprising 10 mM KCI. Suspended in the source solution 141 is 75 µM of the first species 151, 2,2'-(1,2,4,5-tetrazine-3,6-diyl)bis(ethan-1-amine) hydrochloride, which under physiological conditions forms the positively charged compound 2,2'-(1,2,4,5-tetrazine-3,6-diyl)bis(ethan-1-aminium) shown in fig. 6 as K2.

The target solution 142 is an aqueous solution comprising 10 mM KCl. Suspended in the target solution 142 is 75 µM of the second species 152, (S,E)-cyclooct-2-en-1-yl (4-methyl-2-oxo-2H-chromen-7-yl) ethane-1,2-diylbis(methylcarbamate). The second species 152 may be abbreviated as rTCO-DMEDA-4MU, wherein the part representing 4MU (4-Methylumbelliferone) is a fluorophore that becomes active once released.

As seen in Fig. 5, in this example the part representing DMEDA in the second species 152 functions as a self-immolating linker that will break the bond with the part representing 4MU in the second species 152 as the first species 151 binds to the rTCO part of the second species 152, thereby releasing 4MU 153. There may be further possible pairs of species based on tetrazine and trans-cyclooctene (TCO) that could be used to achieve a similar result.

The system 100 is positioned in a fluorescence detection system arranged to excite at 365 nm wavelength and detect emission at 450 nm wavelength. The device 101 is filled with the source solution 141 with the first species 151 and the target solution 142 contains the second species 152. A 1V potential is applied between the electrodes 131,132, in this example the first species is a cation therefore the electric field is from the first electrode 131 to the second electrode 132. An increase in fluorescence intensity is detected in the target solution 142 as 4MU 153 is released from the second part 154 of the second species 152 indicating that the first species 151 is transported into the target solution 142 and interacts with the second species 152.

## Claims

1. A system for releasing a species, the system (100;200) comprises a device (101;201) comprising a body (102;202), an ion conductive member (120;220), a first electrode (131;231), and a second electrode (132;232,233), the system (100;200) further comprises a source solution (141;241), a target solution (142;242,243), a first species (151;251), a second species (152), and an electrical power source (160;260) connected to the first (131;232) and second electrode (132;232,233),
a first end (121;221) of the ion conductive member (120;220) is arranged in contact with the source solution (141;241),
a second end (122;222) of the ion conductive member (120;220) is arranged in contact with the target solution (142;242,243),
the first electrode (131;231) is arranged in contact with the source solution (141;241), the second electrode (132;232,233) is arranged in contact with the target solution (142;242,243),
the first species (151,251) is in the source solution (141;241), and
the second species (152) is in the target solution (142,242,243),
wherein
the ion conductive member (120;220) is configured to, under the influence of an electrical field provided by the electrical power source (160,260), allow transport of the first species (151;251) through the ion conductive member (120;220) from the source solution (141,241) to the target solution (142;242,243),
the first species (151;251) is arranged to interact with the second species (152) in the target solution (142;242,243), such that at least one part (153;153a,153b,153c,153d) of the second species (152) is released, and wherein
the interaction between the first species (151;251) and the second species (152) is a bioorthogonal chemical reaction.

2. The system according to claim 1, wherein the device (101;201) comprises a first vessel (111;211) and a second vessel (112;212,213), wherein the source solution (141;242) is arranged in the first vessel (111;211), and the target solution (142;242,243) is arranged in the second vessel (112;212,213).

3. The system according to any of the preceding claims, wherein the first species (151;251) is arranged to interact with the second species (152) by at least one elimination reaction, thereby releasing at least one part (153;153a,153b,153c,153d) of the second species (152) into the target solution (142;242,243).

4. The system according to any of the preceding claims, wherein the first species (151;251) comprises at least one of:
azide,
phosphine,
palladium compound,
tetrazine,
trans-cyclooctene,
cycloalkyne, or
derivatives of any one thereof.

5. The system according to any of the preceding claims, wherein the second species (152) comprises at least one of:
tetrazine,
trans-cyclooctene,
isonitrile,
4-azidobenzyl carbamate,
propargyl carbamate,
1,2,3,4-tetrazine-3-carboxamide,
mesoionic sydnones,
sulfonyl sydnonimines,
dibenzoazacyclooctyne (DIBAC),
vinyl ether,
benzonorbornadiene, or
derivatives of any one thereof.

6. The system according to any of the preceding claims, wherein the ion conductive member (120;220) is a cation exchange membrane.

7. The system according to any of claims 1-5, wherein the ion conductive member (120;220) is an anion exchange membrane.

8. The system according to any of the preceding claims, comprising two or more target solutions (242,243),
wherein the ion conducting member (220) comprises two or more second ends (222,223), each second end (222,223) arranged in contact with a respective target solution (242,243), and
wherein the ion conductive member (220) is arranged to transport the first species (251) from the source solution (241) to each target solution (242,243).

9. The system according to any of the preceding claims, comprising two or more source solutions (141;241),
wherein the ion conducting member (120;220) comprises two or more first ends (121;221), each first end (121;221) arranged in contact with a respective source solution (141;241), and
wherein the ion conductive member (120;220) is arranged to transport the first species (151;251) from each source solution (141;241) to the target solution (142;242,243).

10. Method for releasing a species, wherein the method (300) comprises the steps of providing (310) a first species (151;251) in a source solution (141;241),
providing (320) a second species (152) in a target solution (142;242,243),
providing (330) an ion conductive member (120;220), wherein a first end (121;221) of the ion conductive member (120;220) is arranged in contact with the source solution (141;241) and a second end (122;222,232) of the ion conductive member (120;220) is arranged in contact with the target solution (142;242,243),
wherein the method further comprises the step of transporting (350) the first species (151;251) into the target solution (142;242,243) via the ion conductive member (120;220) by applying an electric field across the ion conductive member (120;220), wherein
the first species (151;251) transported to the target solution (142;242,243) is arranged to interact with the second species (152), such that at least one part (153;153a,153b,153c,153d) of the second species (152) is released, and
the interaction between the first species (151;251) and the second species (152) is a bioorthogonal chemical reaction.

11. The method according to claim 10, wherein the step of providing (310) a first species (151;251) further comprises providing a fist vessel (111;211) and arranging the source solution (141;241) in said vessel (111;211), and/or
the step of providing (320) a second species (152) further comprises providing a second vessel (112;212,213) and arranging the target solution (142;242,243) in said vessel (112;212,213).

12. A device for releasing a species, the device (101;201) comprises a body (102;202), a first vessel (111;211), a second vessel (112;212,213), an ion conductive member (120;220), a first electrode (131;231), and a second electrode (132;232,233),
the first vessel (111;211) is arranged to accommodate a source solution (141;241),
the second vessel (112;212,213) is arranged to accommodate a target solution (142;242,243),
a first end (121;221) of the ion conductive member (120;220) connects to the first vessel (111;211) and is arranged to be in contact with the source solution (141;241) when accommodated in the first vessel (111;211),
a second end (122;222,223) of the ion conductive member (120;220) connects to the second vessel (112;212,213) and is arranged to be in contact with the target solution (142;242,243) when accommodated in the second vessel (112;212,213),
the first electrode (131;231) is arranged at the first vessel (111;211) and is arranged to be in contact with the source solution (141;241) when accommodated in the first vessel (111;211),
the second electrode (132;232,233) is arranged at the second vessel (112;212,213) and is arranged to be in contact with the target solution (142;242,243) when accommodated in the second vessel (112;212,213),
the first vessel (111;211) is arranged to accommodate a first species (151;251) in the source solution (141;241),
the second vessel (112;212,213) is arranged to accommodate a second species (152) in the target solution (142;242,243), and
the ion conductive member (120;220) is arranged to, under the influence of an applied electrical field allow transport of the first species (151;251) through the ion conductive member (120;220) from the first vessel (111;211) to the second vessel (112;212,213), wherein
the first species (151;251) transported to the second vessel (112;212,213) is arranged to interact with the second species (152), whereby at least one part (153;153a,153b,153c,153d) of said second species (152) is released, and
the interaction between the first species (151;251) and the second species (152) is a bioorthogonal chemical reaction.

## Patentansprüche

1. System zum Freisetzen einer Spezies, wobei das System (100;200) eine Vorrichtung (101;201), umfassend einen Körper (102;202), ein ionenleitendes Element (120;220), eine erste Elektrode (131;231) und eine zweite Elektrode (132;232,233), umfasst, wobei das System (100;200) ferner eine Quelllösung (141;241), eine Ziellösung (142;242,243), eine erste Spezies (151;251), eine zweite Spezies (152) und eine elektrische Stromquelle (160;260), die mit der ersten (131;232) und der zweiten Elektrode (132;232,233) verbunden ist, umfasst,
ein erstes Ende (121;221) des ionenleitenden Elements (120;220) in Kontakt mit der Quelllösung (141;241) angeordnet ist,
ein zweites Ende (122;222) des ionenleitenden Elements (120;220) in Kontakt mit der Ziellösung (142;242,243) angeordnet ist,
die erste Elektrode (131;231) in Kontakt mit der Quelllösung (141;241) angeordnet ist,
die zweite Elektrode (132;232,233) in Kontakt mit der Ziellösung (142;242,243) angeordnet ist,
sich die erste Spezies (151,251) in der Quelllösung (141;241) befindet und
sich die zweite Spezies (152) in der Ziellösung (142,242,243) befindet,
wobei
das ionenleitende Element (120;220) dazu konfiguriert ist, unter dem Einfluss eines elektrischen Felds, das durch die elektrische Stromquelle (160,260) bereitgestellt wird, einen Transport der ersten Spezies (151;251) durch das ionenleitende Element (120;220) von der Quelllösung (141,241) zu der Ziellösung (142;242,243) zuzulassen,
die erste Spezies (151;251) dazu angeordnet ist, mit der zweiten Spezies (152) in der Ziellösung (142;242,243) derart zu interagieren, dass mindestens ein Teil (153;153a,153b,153c,153d) der zweiten Spezies (152) freigesetzt wird, und wobei
die Interaktion zwischen der ersten Spezies (151;251) und der zweiten Spezies (152) eine bioorthogonale chemische Reaktion ist.

2. System nach Anspruch 1, wobei die Vorrichtung (101;201) ein erstes Gefäß (111;211) und ein zweites Gefäß (112;212,213) umfasst, wobei die Quelllösung (141;242) in dem ersten Gefäß (111;211) angeordnet ist und die Ziellösung (142;242,243) in dem zweiten Gefäß (112;212,213) angeordnet ist.

3. System nach einem der vorhergehenden Ansprüche, wobei die erste Spezies (151;251) dazu angeordnet ist, mit der zweiten Spezies (152) durch mindestens eine Eliminierungsreaktion zu interagieren, wodurch mindestens ein Teil (153;153a,153b,153c,153d) der zweiten Spezies (152) in die Ziellösung (142;242,243) freigesetzt wird.

4. System nach einem der vorhergehenden Ansprüche, wobei die erste Spezies (151;251) mindestens eines der Folgenden umfasst:
Azid,
Phosphin,
Palladiumverbindung,
Tetrazin,
Trans-Cycloocten,
Cycloalkin oder
Derivate von einem beliebigen dieser.

5. System nach einem der vorhergehenden Ansprüche, wobei die zweite Spezies (152) mindestens eines der Folgenden umfasst:
Tetrazin,
Trans-Cycloocten,
Isonitril,
4-Azidobenzylcarbamat,
Propargylcarbamat,
1,2,3,4-Tetrazin-3-carboxamid,
mesoionische Sydnone,
Sulfonylsydnonimine,
Dibenzoazacyclooctin (DIBAC),
Vinylether,
Benzonorbornadien oder
Derivate von einem beliebigen dieser.

6. System nach einem der vorhergehenden Ansprüche, wobei das ionenleitende Element (120;220) eine Kationenaustauschmembran ist.

7. System nach einem der Ansprüche 1-5, wobei das ionenleitende Element (120;220) eine Anionenaustauschmembran ist.

8. System nach einem der vorhergehenden Ansprüche, umfassend zwei oder mehr Ziellösungen (242,243),
wobei das ionenleitende Element (220) zwei oder mehr zweite Enden (222,223) umfasst, wobei jedes zweite Ende (222,223) in Kontakt mit einer jeweiligen Ziellösung (242,243) angeordnet ist und
wobei das ionenleitende Element (220) dazu angeordnet ist, die erste Spezies (251) von der Quelllösung (241) zu jeder Ziellösung (242,243) zu transportieren.

9. System nach einem der vorhergehenden Ansprüche, umfassend zwei oder mehr Quelllösungen (141;241),
wobei das ionenleitende Element (120;220) zwei oder mehr erste Enden (121;221) umfasst, wobei jedes erste Ende (121;221) in Kontakt mit einer jeweiligen Quelllösung (141;241) angeordnet ist und
wobei das ionenleitende Element (120;220) dazu angeordnet ist, die erste Spezies (151;251) von jeder Quelllösung (141;241) zu der Ziellösung (142;242,243) zu transportieren.

10. Verfahren zum Freisetzen einer Spezies, wobei das Verfahren (300) die folgenden Schritte umfasst:
Bereitstellen (310) einer ersten Spezies (151;251) in einer Quelllösung (141;241),
Bereitstellen (320) einer zweiten Spezies (152) in einer Ziellösung (142;242,243),
Bereitstellen (330) eines ionenleitenden Elements (120;220), wobei ein erstes Ende (121;221) des ionenleitenden Elements (120;220) in Kontakt mit der Quelllösung (141;241) angeordnet ist und ein zweites Ende (122;222,232) des ionenleitenden Elements (120;220) in Kontakt mit der Ziellösung (142;242,243) angeordnet ist,
wobei das Verfahren ferner den folgenden Schritt umfasst:
Transportieren (350) der ersten Spezies (151;251) über das ionenleitende Element (120;220) in die Ziellösung (142;242,243) durch Anlegen eines elektrischen Felds über das ionenleitende Element (120;220), wobei
die erste Spezies (151;251), die zu der Ziellösung (142;242,243) transportiert wird, dazu angeordnet ist, derart mit der zweiten Spezies (152) zu interagieren, dass mindestens ein Teil (153;153a,153b,153c,153d) der zweiten Spezies (152) freigesetzt wird, und
wobei die Interaktion zwischen der ersten Spezies (151;251) und der zweiten Spezies (152) eine bioorthogonale chemische Reaktion ist.

11. Verfahren nach Anspruch 10, wobei der Schritt des Bereitstellens (310) einer ersten Spezies (151;251) ferner Bereitstellen eines ersten Gefäßes (111;211) und Anordnen der Quelllösung (141;241) in dem Gefäß (111;211) umfasst und/oder der Schritt des Bereitstellens (320) einer zweiten Spezies (152) ferner Bereitstellen eines zweiten Gefäßes (112;212,213) und Anordnen der Ziellösung (142;242,243) in dem Gefäß (112;212,213) umfasst.

12. Vorrichtung zum Freisetzen einer Spezies, wobei die Vorrichtung (101;201) einen Körper (102;202), ein erstes Gefäß (111;211), ein zweites Gefäß (112;212,213), ein ionenleitendes Element (120;220), eine erste Elektrode (131;231) und eine zweite Elektrode (132;232,233) umfasst,
das erste Gefäß (111;211) dazu angeordnet ist, eine Quelllösung (141;241) aufzunehmen, das zweite Gefäß (112;212,213) dazu angeordnet ist, eine Ziellösung (142;242,243) aufzunehmen,
ein erstes Ende (121;221) des ionenleitenden Elements (120;220) mit dem ersten Gefäß (111;211) verbunden ist und dazu angeordnet ist, in Kontakt mit der Quelllösung (141;241) zu stehen, wenn diese in dem ersten Gefäß (111;211) aufgenommen ist,
ein zweites Ende (122;222,223) des ionenleitenden Elements (120;220) mit dem zweiten Gefäß (112;212,213) verbunden ist und dazu angeordnet ist, in Kontakt mit der Ziellösung (142;242,243) zu stehen, wenn diese in dem zweiten Gefäß (112;212,213) aufgenommen ist,
die erste Elektrode (131;231) an dem ersten Gefäß (111;211) angeordnet ist und dazu angeordnet ist, in Kontakt mit der Quelllösung (141;241) zu stehen, wenn diese in dem ersten Gefäß (111;211) aufgenommen ist,
die zweite Elektrode (132;232,233) an dem zweiten Gefäß (112;212,213) angeordnet ist und dazu angeordnet ist, in Kontakt mit der Ziellösung (142;242,243) zu stehen, wenn diese in dem zweiten Gefäß (112;212,213) aufgenommen ist,
das erste Gefäß (111;211) dazu angeordnet ist, eine erste Spezies (151;251) in der Quelllösung (141;241) aufzunehmen,
das zweite Gefäß (112;212,213) dazu angeordnet ist, eine zweite Spezies (152) in der Ziellösung (142;242,243) aufzunehmen, und das ionenleitende Element (120;220) dazu angeordnet ist, unter dem Einfluss eines angelegten elektrischen Felds einen Transport der ersten Spezies (151;251) durch das ionenleitende Element (120;220) von dem ersten Gefäß (111;211) zu dem zweiten Gefäß (112;212,213) zuzulassen, wobei
die erste Spezies (151;251), die in das zweite Gefäß (112;212,213) transportiert wird, dazu angeordnet ist, mit der zweiten Spezies (152) zu interagieren, wodurch mindestens ein Teil (153;153a,153b,153c,153d) der zweiten Spezies (152) freigesetzt wird, und
die Interaktion zwischen der ersten Spezies (151;251) und der zweiten Spezies (152) eine bioorthogonale chemische Reaktion ist.

## Revendications

1. Système de libération d'une espèce, le système (100;200) comprenant un dispositif (101;201) comprenant un corps (102;202),un élément conducteur d'ions (120;220),une première électrode (131;231) et une seconde électrode (132;232,233),le système (100;200) comprenant en outre une solution source (141;241),une solution cible (142;242,243),une première espèce (151;251),une seconde espèce (152) et une source d'alimentation électrique (160;260) connectée à la première (131;232) et à la seconde électrode (132;232,233),
une première extrémité (121;221) de l'élément conducteur d'ions (120;220) est disposée en contact avec la solution source (141;241),
une seconde extrémité (122;222) de l'élément conducteur d'ions (120;220) est disposée en contact avec la solution cible (142;242,243),
la première électrode (131;231) est disposée en contact avec la solution source (141;241),
la seconde électrode (132;232,233) est disposée en contact avec la solution cible (142;242,243),
la première espèce (151,251) se trouve dans la solution source (141;241),et
la seconde espèce (152) se trouve dans la solution cible (142,242,243),
dans lequel
l'élément conducteur d'ions (120;220) est configuré pour, sous l'influence d'un champ électrique fourni par la source d'alimentation électrique (160,260),permettre le transport de la première espèce (151;251) à travers l'élément conducteur d'ions (120;220) de la solution source (141,241) à la solution cible (142;242,243),
la première espèce (151;251) est agencée pour interagir avec la seconde espèce (152) dans la solution cible (142;242,243),de telle sorte qu'au moins une partie (153;153a,153b,153c,153d) de la seconde espèce (152) est libérée, et dans lequel
l'interaction entre la première espèce (151;251) et la seconde espèce (152) est une réaction chimique bioorthogonale.

2. Système selon la revendication 1,dans lequel le dispositif (101;201) comprend un premier récipient (111;211) et un second récipient (112;212,213),dans lequel la solution source (141;242) est disposée dans le premier récipient (111;211),et la solution cible (142;242,243) est disposée dans le second récipient (112;212,213).

3. Système selon l'une quelconque des revendications précédentes, dans lequel la première espèce (151;251) est agencée pour interagir avec la seconde espèce (152) par au moins une réaction d'élimination, libérant ainsi au moins une partie (153;153a,153b,153c,153d) de la seconde espèce (152) dans la solution cible (142;242,243).

4. Système selon l'une quelconque des revendications précédentes, dans lequel la première espèce (151;251) comprend au moins l'un des éléments suivants :
l'azoture,
la phosphine,
un composé de palladium,
le tétrazine,
le trans-cyclooctène,
le cycloalcyne,ou
des dérivés de l'un quelconque de ceux-ci.

5. Système selon l'une quelconque des revendications précédentes, dans lequel la seconde espèce (152) comprend au moins l'un des éléments suivants :
le tétrazine,
le trans-cyclooctène,
l'isonitrile,
le carbamate de 4-azidobenzyle,
le carbamate de propargyle,
1,2,3,4-tétrazine-3-carboxamide,
les sydnones mésoioniques,
les sydnonimines sulfonyle,
le dibenzoazacyclooctyne (DIBAC),
le vinyle éther,
le benzonorbornadiène, ou
des dérivés de l'un quelconque de ceux-ci.

6. Système selon l'une quelconque des revendications précédentes, dans lequel l'élément conducteur d'ions (120;220) est une membrane échangeuse de cations.

7. Système selon l'une quelconque des revendications 1 à 5, dans lequel l'élément conducteur d'ions (120;220) est une membrane échangeuse d'anions.

8. Système selon l'une quelconque des revendications précédentes, comprenant deux ou plusieurs solutions cibles (242,243),
dans lequel l'élément conducteur d'ions (220) comprend deux ou plusieurs secondes extrémités (222,223), chaque seconde extrémité (222,223) étant disposée en contact avec une solution cible respective (242,243), et
dans lequel l'élément conducteur d'ions (220) est agencé pour transporter la première espèce (251) de la solution source (241) vers chaque solution cible (242,243).

9. Système selon l'une quelconque des revendications précédentes, comprenant deux ou plusieurs solutions sources (141;241),
dans lequel l'élément conducteur d'ions (120;220) comprend deux ou plusieurs premières extrémités (121;221),chaque première extrémité (121;221) étant disposée en contact avec une solution source respective (141;241), et
dans lequel l'élément conducteur d'ions (120;220) est agencé pour transporter la première espèce (151;251) de chaque solution source (141;241) vers la solution cible (142;242,243).

10. Procédé de libération d'une espèce, dans lequel le procédé (300) comprend les étapes consistant à
fournir (310) une première espèce (151;251) dans une solution source (141;241),
fournir (320) une seconde espèce (152) dans une solution cible (142;242,243),
fournir (330) un élément conducteur d'ions (120;220),dans lequel une première extrémité (121;221) de l'élément conducteur d'ions (120;220) est disposée en contact avec la solution source (141; 241) et une seconde extrémité (122;222,232) de l'élément conducteur d'ions (120;220) est disposée en contact avec la solution cible (142;242,243),
dans lequel le procédé comprend en outre l'étape consistant à transporter (350) la première espèce (151;251) dans la solution cible (142;242,243) via l'élément conducteur d'ions (120;220) en appliquant un champ électrique à travers l'élément conducteur d'ions (120;220),dans lequel
la première espèce (151;251) transportée vers la solution cible (142;242,243) est agencée pour interagir avec la seconde espèce (152),de telle sorte qu'au moins une partie (153;153a,153b,153c,153d) de la seconde espèce (152) est libérée, et
l'interaction entre la première espèce (151;251) et la seconde espèce (152) est une réaction chimique bioorthogonale.

11. Procédé selon la revendication 10,dans lequel l'étape de fourniture (310) d'une première espèce (151;251) comprend en outre la fourniture d'un premier récipient (111;211) et la disposition de la solution source (141;241) dans ledit récipient (111;211),et/ou
l'étape de fourniture (320) d'une seconde espèce (152) comprend en outre la fourniture d'un second récipient (112;212,213) et la disposition de la solution cible (142;242,243) dans ledit récipient (112;212,213).

12. Dispositif de libération d'une espèce, le dispositif (101;201) comprenant un corps (102;202),un premier récipient (111;211),un second récipient (112;212,213),un élément conducteur d'ions (120;220),une première électrode (131;231) et une seconde électrode (132;232,233),
le premier récipient (111;211) est agencé pour recevoir une solution source (141;241),le second récipient (112;212,213) est agencé pour recevoir une solution cible (142;242,243),
une première extrémité (121;221) de l'élément conducteur d'ions (120;220) se connecte au premier récipient (111;211) et est disposée pour être en contact avec la solution source (141;241) lorsqu'elle est logée dans le premier récipient (111;211),
une seconde extrémité (122;222,223) de l'élément conducteur d'ions (120;220) se connecte au second récipient (112;212,213) et est disposée pour être en contact avec la solution cible (142;242,243) lorsqu'elle est logée dans le second récipient (112;212,213),
la première électrode (131;231) est disposée au niveau du premier récipient (111;211) et est disposée pour être en contact avec la solution source (141;241) lorsqu'elle est logée dans le premier récipient (111;211),
la seconde électrode (132;232,233) est disposée au niveau du second récipient (112;212,213) et est disposée pour être en contact avec la solution cible (142;242,243) lorsqu'elle est logée dans le second récipient (112;212,213),
le premier récipient (111;211) est agencé pour accueillir une première espèce (151;251) dans la solution source (141;241),
le second récipient (112;212,213) est agencé pour accueillir une seconde espèce (152) dans la solution cible (142;242,243), et
l'élément conducteur d'ions (120;220) est conçu pour, sous l'influence d'un champ électrique appliqué, permettre le transport de la première espèce (151;251) à travers l'élément conducteur d'ions (120;220) du premier récipient (111;211) au second récipient (112;212,213), dans lequel
la première espèce (151;251) transportée vers le second récipient (112;212,213) est agencée pour interagir avec la seconde espèce (152),moyennant quoi au moins une partie (153;153a,153b,153c,153d) de ladite seconde espèce (152) est libérée, et
l'interaction entre la première espèce (151;251) et la seconde espèce (152) est une réaction chimique bioorthogonale.
